(19) Europäisches Patentamt
European Patent Office
Office européen des brevets

(11) Veröffentlichungsnummer: **0 428 854 A2**

# (12) EUROPÄISCHE PATENTANMELDUNG

(21) Anmeldenummer: **90119217.9**

(22) Anmeldetag: **06.10.90**

(51) Int. Cl.5: **C07C 47/228**, C07C 45/41, C07C 67/343, C07C 69/738, C07C 67/317, C07C 69/612, C07D 495/14, A61K 31/55, //(C07D495/14,333:00,249:00, 243:00)

(30) Priorität: **10.10.89 DE 3933781**
**04.11.89 DE 3936766**

(43) Veröffentlichungstag der Anmeldung:
**29.05.91 Patentblatt 91/22**

(84) Benannte Vertragsstaaten:
**AT BE CH DE DK ES FR GB GR IT LI LU NL SE**

(71) Anmelder: **BOEHRINGER INGELHEIM KG**
**Postfach 200**
**W-6507 Ingelheim am Rhein(DE)**
(84) **BE CH DE DK ES FR GR IT LI LU NL SE AT**

Anmelder: **BOEHRINGER INGELHEIM INTERNATIONAL GmbH**
**Postfach 20**
**W-6507 Ingelheim am Rhein(DE)**
(84) **GB**

(72) Erfinder: **Stransky, Werner, Dr.**
**Im Hippel 24**
**W-6535 Gau-Algesheim(DE)**
Erfinder: **Brandt, Klaus, Dr.**
**Georg-Scheuing-Strasse 22**
**W-6507 Ingelheim am Rhein(DE)**
Erfinder: **Knorr, Hansjörg**
**Waldstrasse 39**
**W-6507 Ingelheim am Rhein(DE)**
Erfinder: **Birke, Franz, Dr.**
**Albrecht-Dürer-Strasse 23**
**W-6507 Ingelheim am Rhein(DE)**
Erfinder: **Heuer, Hubert, Dr.**
**Am Sportfeld 74**
**W-6501 Schwabenheim(DE)**

## (54) Verfahren zur Herstellung von 4-(4-Isobutylphenyl)-butyraldehyd und seine Verwendung zur Herstellung von (-)-4-(2-Chlorphenyl)-2-[2-(4-isobutylphenyl)-ethyl]-6,9-dimethyl-6H-thieno-[3,2-f][1,2,4]-triazolo[4,3-a][1,4]diazepin.

(57) Die Erfindung betrifft eine einfache, wirtschaftliche Synthese von 4-(4-Isobutylphenyl)-butyraldehyd und seine Verwendung zur Herstellung von (-)-4-(2-Chlorphenyl)-2-[2-(4-isobutylphenyl)ethyl]-6,9-dimethyl-6H-thieno-[3,2-f][1,2,4]triazolo[4,3-a]-1,4-diazepin.

EP 0 428 854 A2

# VERFAHREN ZUR HERSTELLUNG VON 4-(4-ISOBUTYLPHENYL)-BUTYRALDEHYD UND SEINE VERWENDUNG ZUR HERSTELLUNG VON (-)-4-(2-CHLORPHENYL)-2-[2-(4-ISOBUTYLPHENYL)-ETHYL]-6,9-DIMETHYL-6H-THIENO[3,2-F][1.2.4]-TRIAZOLO[4,3-A][1,4]DIAZEPIN

Die Erfindung betrifft eine einfache, wirtschaftliche Synthese von 4-(4-Isobutylphenyl)-butyraldehyd.

Aldehyde des oben genannten Strukturtyps sind wichtige Ausgangsverbindungen zur Synthese von substituierten 6H-Thieno[3,2-f][1,2,4]triazolo[4,3-a][1,4]diazepinen, die als Arzneimittel von Bedeutung sind. 4-(4-Isobutylphenyl)butyraldehyd ist namentlich erstmals in der europäischen Patentanmeldung 268 242 erwähnt, jedoch enthält die Patentanmeldung weder physikalische Daten dieser Verbindung, noch wird offenbart wie dieser Aldehyd hergestellt wurde. Es ist demnach die Aufgabe der vorliegenden Erfindung ein einfaches, möglichst wenig Synthesestufen umfassendes Herstellverfahren für den 4-(4-Isobutylphenyl)-butyraldehyd vorzuschlagen.

Die Aufgabe der vorliegenden Erfindung wird wie folgt gelöst:

Ausgehend von Isobutylbenzol erhält man durch Umsetzung mit Bernsteinsäuremethylesterchlorid den 4-(4-Isobutylphenyl)-4-oxo-buttersäuremethylester. Dieser wird im nachfolgenden Reaktionsschritt katalytisch zu dem 4-(4-Isobutylphenyl)-buttersäure-methylester reduziert. Das gewünschte Endprodukt erhält man durch anschließende Reduktion des Esters mit einem geeigneten metallorganischen Reduktionsmittel -insbesondere mit Diisobutylaluminiumhydrid. Die beanspruchte Reaktionsfolge ist im Syntheseschema I wiedergegeben.

Anstelle der oben beschriebenen Methylester können auch die anderen entsprechenden $C_1$-$C_4$-Alkylester in das Verfahren eingesetzt werden.

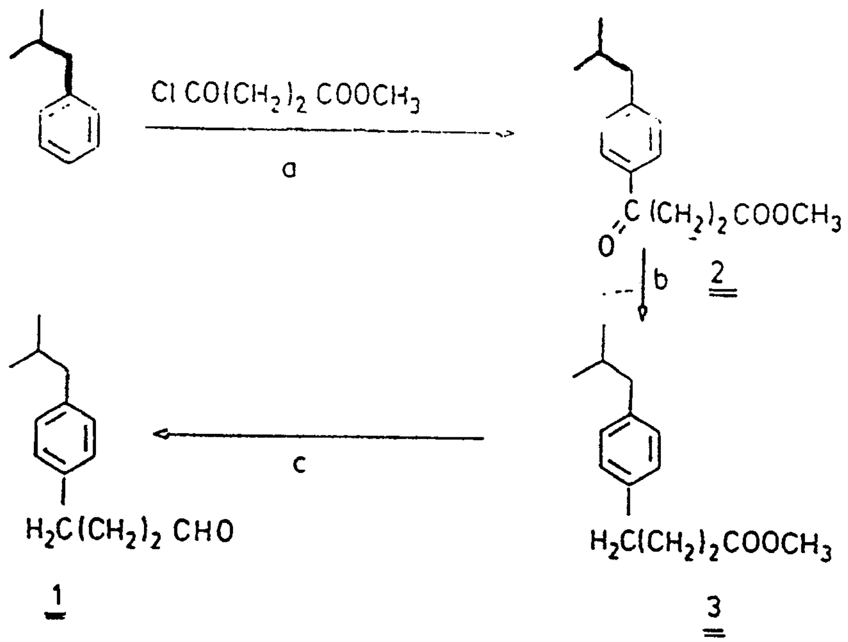


Die Friedel-Crafts-Acylierung (a) erfolgt in aprotischen Lösungsmitteln, wie z.B. Nitromethan, Dichlormethan, Trichlormethan; bevorzugtes Lösungsmittel ist Schwefelkohlenstoff. Als Katalysatoren können Lewis-Säuren - wie z.B. $FeCl_3$, $TiCl_4$ und $ZnCl_2$ - eingesetzt werden; bevorzugter Katalysator ist $AlCl_3$. Sinnvollerweise wird die Umsetzung in einem Temperaturbereich zwischen 10 °C und 40 °C, bevorzugt zwischen 10 °C und 20 °C durchgeführt, da in diesem Temperaturbereich ausschließlich die gewünschte para-Substitution erfolgt. Eine ortho-Substitution konnte NMR-spektroskopisch überraschenderweise nicht nachgewiesen werden. Die katalytische Hydrierung von (2) erfolgt in Essigsäure in Gegenwart von Pd/Kohle oder Pt-Kohle in einem Autoklaven bei erhöhten Drucken. Vorzugsweise wird die Hydrierung bei Raumtemperatur durchgeführt, da bei erhöhten Temperaturen zumindest mit einer teilweisen Reduktion des Phenylringes zu rechnen ist.

Die anschließende Reduktion des Esters (3) erfolgt nach an sich bekannten Standardverfahren z.B. mit Diisobutylaluminiumhydrid in einem inerten Lösungsmittel, wie z.B. Toluol. Der nach den oben beschriebenen Verfahren hergestellte Aldehyd eignet sich als Ausgangsverbindung zur Herstellung nachfolgend genannter Hetrazepine.

a) 4-(2-Chlorphenyl)-2-[2-(4-isobutylphenyl)ethyl]-9-methyl-6H-thieno[3,2-f][1,2,4]triazolo[4,3-a][1,4]-diazepin

b) 4-(2-Chlorphenyl)-2-[2-(4-isobutylphenyl)ethyl]-6,9-dimethyl-thieno-[3,2-f][1,2,4]triazolo[4,3-a][1,4]-diazepin.

Ausgehend von dem 4-(4-Isobutylphenyl)-butyraldehyd erfolgt die Synthese von a in Analogie zu dem in der europäischen Patentanmeldung 268 242 beschriebenen Verfahren, wobei folgende Zwischenverbindungen isoliert werden.

1) 2-Amino-3-(2-chlorbenzoyl)-5-[2-(4-isobutylphenyl)ethyl]thiophen
2) 2-Bromacetylamino-3-(2-chlorbenzoyl)-5-[2-(4-isobutylphenyl)ethyl]thiophen
3) 2-Aminoacetylamino-3-(2-chlorbenzoyl)-5-[2-(4-isobutylphenyl)ethyl]thiophen
4) 5-(2-Chlorphenyl)-7-[2-(4-isobutylphenyl)ethyl]-1H-2,3-dihydro-thieno[2,3-e]-1,4-diazepin-2-on
5) 5-(2-Chlorphenyl)-7-[2-(4-isobutylphenyl)ethyl]-1H-2,3-dihydro-thieno[2,3-e]-1,4-diazepin-2-thion
6) 5-(2-Chlorphenyl)-7-[2-(4-isobutylphenylethoxy]-1H-2,3-dihydro-thieno-[2,3-e]-1,4-diazepin-2-hydrazon

Das Hydrazon (6) wird dann mit Essigsäureanhydrid/Essigsäure in Toluol in die Verbindung (a) von Schmelzpunkt 118 - 121 °C überführt.

In Analogie zu den oben angegebenen Syntheseschritten wird das 4-(2-Chlorphenyl)-2-[2-(4-isobutylphenyl)ethyl]6,9-dimethylthieno[3,2-f][1,2,4]triazolo[4,3-a][1,4]-diazepin (b) hergestellt, wobei jedoch das entsprechende 2-Amino-3-(2-chlorbenzoyl)-5-[2-(4-isobutylphenyl)ethyl]thiophen mit Chlorpropionylchlorid zu dem 3-(2-Chlorbenzoyl)-2-(2-chlorpropionylamino)-5-[2-(4-isobutylphenyl)ethyl]thiophen umgesetzt wird; anschließend erfolgt ein Halogenaustausch gegen Jod. Das Jod-Derivat wird dann in das 2-(2-Aminopropionylamino)-3-(2-chlorbenzoyl)-5-[2-(4-isobutylphenyl)ethyl]thiophen überführt. Weitere Zwischenverbindungen, die erhalten werden, sind:
5-(2-Chlorphenyl)-3-methyl-7-[2-(4-isobutylphenyl)ethyl]-1H-2,3-dihydro-thieno[2,3-e]-1,4-diazepin-2-on und das 5-(2-Chlorphenyl)-3-methyl-7-[2-(4-isobutylphenyl)ethyl]-1H-2,3-dihydro-thieno[2,3-e]-1,4-diazepin-2-thion, welches anschließend mit Hydrazinhydrat zu dem 5-(2-Chlorphenyl)-3-methyl-7-[2-(4-isobutylphenyl)-ethyl]-1H-2,3-dihydro-thieno[2,3-e]-1,4-diazepin-2-hydrazon umgesetzt wird.

Dieses wird in Analogie zu ebenfalls bekannten Verfahren mit Acetanhydrid/Eisessig in die Titelverbindung (b) vom Schmelzpunkt 129,5 - 131,5 °C überführt.

Das 4-(2-Chlorphenyl)-2-[2-(4-isobutylphenyl)ethyl]-6,9-dimethyl-6H-thieno[3,2-f][1,2,4]triazolo[4,3-a]-[1,4]diazepin besitzt am C-6-Kohlenstoffatom ein Asymmetriezentrum und liegt zunächst als Racemat vor, das in seine Enantiomere aufgetrennt werden kann.

Die Trennung erfolgt nach folgender Methode:
100 mg des Racemats werden auf einer semipräparativen Säule (250 mm x 10 mm i.D.), welche mit Poly-N-acryloyl-L-phenylalaninethylester gefüllt ist, in die Enantiomeren getrennt.
Als mobile Phase wird ein Gemisch von n-Hexan und Dioxan (60/40 v/v) benutzt. Der Durchfluß beträgt 5 ml/min, die Meßwellenlänge 254nm, die Temperatur 20 °C. Es werden pro Injektion 10 mg des Racemats, gelöst in mobiler Phase, auf die Säule aufgegeben und die getrennten Enantiomere einzeln isoliert. Die optische Reinheit der isolierten Enantiomeren wird anschließend auf einer analytischen Säule, gefüllt mit der gleichen Trennphase, kontrolliert (Durchlaß: 1 ml/min). Die getrennten Enantiomere werden nochmals auf der semipräparativen Säule nachgereinigt und erneut auf der analytischen Säule ihre optische Reinheit (ee) bestimmt. Es wurden von beiden Antipoden jeweils 30 mg mit einer optischen Reinheit von > 99 % erhalten. Die spezifische Drehung beider Komponenten wurde gemessen:

Man erhält das
(+)-4-(2-Chlorphenyl)-2-[2-(4-isobutylphenyl)ethyl]-6,9-dimethyl-6H-thieno[3,2-f][1,2,4]triazolo[4,3-a][1,4]-diazepin ((+)-Enantiomer) mit einem Drehwert $[\alpha]_D20$ = +79,1 ° (c = 3,63, Methanol) und das
(-)-4-(2-Chlorphenyl)-2-[2-(4-isobutylphenyl)ethyl]-6,9-dimethyl-6H-thieno[3,2-f][1,2,4]triazolo[4,3-a][1,4]-diazepin ((-)-Enantiomer) mit einem Drehwert $[\alpha]_D20$ = -77,6 ° (c = 3,84, Methanol).

Überraschenderweise wurde gefunden, daß das (-)-Enantiomere 40-mal wirkungsstärker ist. Die pharmakologische Wirkung wurde anhand des nachfolgenden Experiments bestimmt:
$[^3H]$PAF-Rezeptor-Bindung an vitalen humanen Blutplättchen

Bestimmt wird die konkurrierende Wechselwirkung von Prüfsubstanzen (hier PAF-Antagonisten) mit der bekannten Wechselwirkung des Radioliganden $[^3H]$PAF zum gleichen Rezeptor.
Die Bindungsstudien wurden an vitalem menschlichen Thrombozyten vorgenommen. Blutproben von gesunden Spendern wurden mit ACD-Puffer verdünnt und zentrifugiert (15 Min., 160 x g). Das blättchenreiche Plasma wird durch Chromatographie an Sepharose CL-2B [Zum Eluieren: HEPES-Puffer, pH 7,4 20 °C] gereinigt.

Definierte Mengen [z.B. 800 µl] der Blättchensuspension wurden 90 Min. bei Raumtemperatur inkubiert, d.h. vermischt mit:

a) einer 30 mmolaren [$^3$H]PAF-Lösung, verdünnt mit Puffer.

b) mit einer 30 pmolaren [$^3$H] PAF-Lösung, die gleichzeitig eine µmolare (nicht markierte) PAF-Lösung enthält,

c) mit der 30 pmolaren [$^3$H]PAF-Lösung und den Lösungen der Prüfsubstanzen (unterschiedliche Konzentrationen)

a) Dient zur Ermittlung der totalen Bindung,

b) dient zur Ermittlung der unspezifischen Bindung.

Die Reaktion wurde durch Vakuumfiltration abgestoppt. Die Filter mit den Blutplättchen werden mit Szintilationsflüssigkeit versetzt und die verbliebene Radioaktivität in einem Counter gemessen. Die spezifische Bindung ergibt sich aus der Differenz zwischen dem Wert für die totale Bindung und dem Wert für die unspezifische Bindung. Man bestimmt entweder die $IC_{50}$-Werte (d.h. jene Konzentration der Prüfsubstanz, die 50 % des Radioliganden - hier [$^3$H]-PAF - vom Rezeptor verdrängt), und gibt diese an -oder errechnet hieraus die $K_i$-Werte. Dies kann computerunterstützt durch ein Iterationsverfahren der Bindungskurven geschehen.

$IC_{50}$- bzw. Ki-Werte sind ein Maß für die Rezeptoraffinität der Testsubstanz. Kleinere Werte zeigen die höhere Affinität an.

| | [$^3$H]PAF-Bindung ($K_i$) [mol/l] |
|---|---|
| Racemat | $8{,}3 \times 10^{-10}$ |
| (-)Enantiomer | $2{,}9 \times 10^{-10}$ |
| (+)Enantiomer | $1{,}2 \times 10^{-8}$ |

Damit sind pharmazeutische Zubereitungen - z.B. in Form von Tabletten, Lösungen, Inhalationspulvern - die ausschließlich das (-)-Enantiomer als Wirkstoffkomponente enthalten gegenüber solchen, die das Racemat enthalten bevorzugt.

In Analogie zu den in der europäischen Patentanmeldung 230 942 beschriebenen Verfahren können aus dem 4-(4-Isobutylphenyl)-butyraldehyd auch folgende Verbindungen synthetisiert werden:
4-(2-Chlorphenyl)-2-[2-(4-isobutylphenyl)ethyl]-9-methyl-6H-thieno[3,2-f]imidazo[1,2-a][1,4]diazepin,
4-(2-Chlorphenyl)-2-[2-(4-isobutylphenyl)ethyl]-6,9-dimethyl-6H-thieno[3,2-f]imidazo[1,2-a][1,4]diazepin,
(+)-4-(2-Chlorphenyl)-2-[2-(4-isobutylphenyl)ethyl]-6,9-dimethyl-6H-thieno[3,2-f]imidazo[1,2-a][1,4]diazepin,
(-)-4-(2-Chlorphenyl)-2-[2-(4-isobutylphenyl)ethyl]-6,9-dimethyl-6H-thieno[3,2-f]imidazo[1,2-a][1,4]diazepin.

Die genannten Verbindungen sind als PAF-Antagonisten zur Behandlung von Krankheiten geeignet, an denen PAF beteiligt ist.

Die nachfolgenden Versuche sollen das erfindungsgemäße Verfahren erläutern.

4-(4-Isobutylohenyl)-4-oxo-buttersäuremethylester

120 g (0.9 mol) Aluminiumchlorid werden in 200 ml Schwefelkohlenstoff suspendiert und ein Gemisch von 120.8 g (0.9 mol) Isobutylbenzol und 137 g (0.9 mol) Bernsteinsäuremethylesterchlorid bei Raumtemperatur zugetropft. Nach vierstündigem Rühren gießt man das Reaktionsgemisch auf Eis, säuert mit 6N Salzsäure an und extrahiert mit Ether. Nach dem Trocknen und Abziehen des Solvens erhält man nach der Destillation des Rückstanden i.Vak. 97.5 g des Ketoesters vom Kp $_{0.2\ Torr}$ = 145 - 150 °C (Ausbeute 44 %). $^1$H-NMR ($CDCl_3$; 250 MHz): $\delta$ = 7.89, 7.22 (4H, 2d, J = 8.0 Hz, Aryl-H); 3.70 (3H, s, $OCH_3$); 3.30 (2H, t, J = 6.0 Hz, CO-$CH_2$); 2.76 (2H, t, J = 6.0 Hz, $OCOCH_2$); 2.53 (2H, d, J = 6,5 Hz, $\underline{CH_2}$-CH); 1.90 (1H, m, $\underline{CH}$-$CH_2$); 0.90 (6H, d, J = 6.5 Hz, $\underline{CH_3}$-CH).

4-(4-Isobutylohenyl)-buttersäuremethylester (2)

87 g (0.35 mol) Oxoester 1, gelöst in 1920 ml Essigsäure und 15 g Pd-Kohle (10%) werden in einem Autoklaven bei 20 °C und 5 bar $H_2$-Druck innerhalb von 45 Minuten hydriert. Nach dem Absaugen des Hydrierkatalysators zieht man die Essigsäure vom Filtrat ab, nimmt den Rückstand in Methylenchlorid auf,

wäscht mit Soda neutral, trocknet und zieht anschließend das Solvens ab. Man erhält 72.8 g Ester (Ausbeute 89 %) als helles Oel, das ohne weitere Aufreinigung umgesetzt wird.
$^1$H-NMR ($CDCl_3$; 250 MHz): δ = 7.06 (4H, m, Aryl-H); 3.65 (3H, s, $OCH_3$); 2.61 (2H, t, J = 7Hz, Phenyl-$CH_2$); 2.43 (2H, d, J - 6Hz, $CH_2$-CH); 2.33 (2H, t, J = 7Hz, CO-$CH_2$); 1.94 (2H, m, Phenyl-$CH_2CH_2$); 1.83 (1H, m, CH-$CH_2$); 0.89 (6H, d, J = 6.5 Hz, $CH_3$-CH).

4-(4-Isobutylphenyl)butyraldehyd (3)

Man löst 72.8 g (0.31 mol) des Esters 2 in 1.4 l absolutem Toluol und tropft unter Stickstoffschutz 210 ml DIBAH, 35%ig in Toluol, (0.44 mol) in 35 Minuten bei -70 °C zu, rührt bei -70 °C eine Stunde weiter und tropft anschließend ebenfalls bei -70 °C vorsichtig 100 ml Methanol zu. Das Reaktionsgemisch wird mit 100 ml gesättigter Diammoniumtartratlösung und ca. 300 ml 2N Salzsäure zersetzt, der Niederschlag abgesaugt und das Filtrat extraktiv mit Toluol aufgearbeitet. Nach dem Abziehen des Toluols erhält man 57.4 g des Aldehyds als farbloses Oel (Ausbeute 90 %).
$^1$H-NMR ($CDCl_3$; 250 MHz): δ = 9.76 (1H, t, J = 1.5 Hz, CH=O); 7.06 (4H, d, Aryl-H); 2.61 (2H, t, J = 7Hz, Phenyl-$CH_2$); 2.44 (2H, s, J = 6Hz, $CH_2$-CH); 2.43 (2H, 2t, J = 7; 1.5 Hz, CO-$CH_2$); 1.94 (2H, m, Phenyl-$CH_2CH_2$); 1.83 (1H, m, CH-$CH_2$); 0.89 (6H, d, J = 6.5 Hz, $CH_3$-CH).

Im folgenden sind die $^1$H-Kernresonanz-spektroskopischen Daten ausgewählter Produkte und Zwischenprodukte aufgeführt:

3-(2 Chlorbenzoyl)-2-(2-chlorpropionylamino)-5-[2-(4-isobutylphenyl)ethyl]thiophen
250 MHz-$^1$H NMR ($CDCl_3$): δ =
12.70 (1H, s, NH), 7.26 - 7.49 (4H, m, Aryl-H), 7.02 (4H, m, Aryl-H), 6.35 (1H, s, Thiophen H), 4.66 (1H, q, J = 7Hz, CH),
2.81 - 3.01 (4H, m, 2 x $CH_2$), 2.42 (2H, d, J = 7 Hz, $CH_2$), 1.87 (3H, d, J = 7 Hz, -$CH_3$), 1.81 (1H, m, CH), 0.88 (6H, d, J = 6.7 Hz, 2 x $CH_3$).

3-(2-Chlorbenzoyl)-2-(2-iodpropionylamino)-5-[2-(4-isobutylphenyl)ethyl]thiophen
250 MHz-$^1$H-NMR ($CDCl_3$): δ =
12.25 (1H, s, NH), 7.27 - 7.48 (4H, m, Aryl-H), 7.02 (4H, s, Aryl-H), 6.33 (1H, s, Thiophen-H), 4.76 (1H, q, J = 7 Hz, CH) 2.80 - 3.00 (4H, m, 2 x -$CH_2$-),
2.42 (2H, d, J = 7 Hz, -$CH_2$-), 2.13 (3H, d, J = 7 Hz -$CH_3$),
1.84 (1H, m, CH), 0.88 (6H, d, J = 6.7 Hz, 2 x -$CH_3$).

2 (2-Aminopropionylamino)-3-(2-chlorbenzoyl)-5-[2-(4-isobutylphenyl)ethyl]thiophen
250 MHz-$^1$H-NMR ($CDCl_3$): δ =
12.88 (1H, s, NH), 7.24 - 7.48 (4H, m, Aryl-H), 7.02 (4H, s, Aryl-H), 6.31 (1H, s, Thiophen-H), 3.75 (1H, q, J = 7 Hz, CH), 2.80 - 2.99 (4H, m, 2 x -$CH_2$-),
2.42 (2H, d, J = 7Hz, -$CH_2$-), 2.19 (2H, s, $NH_2$), 1.82 (1H, m, CH), 1.48 (3H, d, J = 7 Hz, -$CH_3$), 0.88 (6H, d, 6.7 Hz, 2 x -$CH_3$).

5-(2-Chlorphenyl)-3-methyl-7-[2-(4-isobutylphenyl) ethyl]-1H-2,3-dihydro-thieno[2,3-e]-1,4-diazepin-2-on
250 MHz-$^1$H-NMR ($CDCl_3$): δ =
8.50 (1H, s, NH), 7.28 - 7.42 (4H, m, Aryl-H), 7.03 (4H, s, Aryl-H), 6.19 (1H, s, Thiophen-H), 3.94 (1H, q, J = 7 Hz, CH), 2.79 - 2.99 (4H, m, 2 x $CH_2$),
2.43 (2H, d, J = 7 Hz, -$CH_2$-), 1.81 (1H, m, CH), 1.77 (3H, d, J = 7 Hz, -$CH_3$), 0.88 (6H, d, J = 6.7 Hz, 2 x $CH_3$).

4-(2 Chlorphenyl-2-[2-(4-isobutylphenyl)ethyl]-6,9-dimethyl-6H-thieno[3,2-f][1,2,4]triazolo[4,3-a][1,4]-diazepin
250 MHz-$^1$H-NMR ($CDCl_3$): δ =
7.28 - 7.48 (4H, m, Aryl-H), 7.28 (4H, s, Aryl-H), 6.36 (1H, s, Thiophen-H), 4.32 (1H, q, J = 7Hz, CH), 2.90 (2H, m, $CH_2$), 3.05 (2H, m, -$CH_2$-), 2.63 (3H, s, -$CH_3$),
2.43 (2H, d J = 7 Hz, -$CH_2$-), 2.09 (3H, d, J = 7 Hz, -$CH_3$),
1.83 (lH, m, CH), 0.88 (6H, d, J = 6.7 Hz, 2 x -$CH_3$).

4-(2-Chlorphenyl)-2-[2-(4-isobutylphenyl)-ethyl]-9-methyl-6H-thieno[3,2-f]imidazo[1,2-a][1,4]diazepin,
$^1$H-NMR ($CDCl_3$): δ = 7.49 - 7.22 (4H, m, o-di-subst.aryl); 7.03 (4H, s, p-subst. aryl); 6.88 ($^1$H, q, J = 1Hz, CH-imidazol); 6.32 (1H, s, CH-thiophen); 4.76 (2H, s, broad, $CH_2$-7-ring); 2.96 (4H, m, $CH_2CH_2$); 2.43 (2H, d, J = 7Hz, $CH_2$-CH); 2.39 (3H, d, J = 1Hz, $CH_3$-imidazol); 1.83 (1H, m, $CH_2$CH); 0.89 (6H, d, J = 6Hz, $(CH_3)_2$-CH).

4-(2-Chlorphenyl)-2-[2-(4-isobutylphenyl)-ethyl]-6,9-dimethyl-6H-thieno[3,2-f]imidazo[1,2a][1,4]diazepin,
$^1$H-NMR ($CDCl_3$): δ = 7.47 - 7.25 (4H, m, o-di-subst. aryl); 7.03 (4H, s, p-di-subst. aryl); 6.90 (1H, q, J =

1Hz, CH-imidazol); 6.34 (1H, s, CH-thiophen); 4.15 (1H, q, J = 6Hz, CH-7-ring); 2.96 (4H, m, $CH_2CH_2$); 2.43 (2H, d, J = 7Hz, $CH_2$-CH); 2.38 (3H, d, J = 1Hz, $CH_3$-imidazol); 2.03 (3H, d, J = 7Hz, $CH_3$-7-ring); 1.82 (1H, m, $CH_2$-CH); 0.88 (6H, d, J = 6Hz, $(CH_3)_2$-CH).

## Ansprüche

1) Verfahren zur Herstellung von 4-(4-Isobutylphenyl)butyraldehyd, dadurch gekennzeichnet, daß man
a) Isobutylbenzol mit einem Bernsteinsäurealkylesterchlorid zu 4-(4-Isobutylphenyl)-4-oxobuttersäurealkylester umsetzt,
b) den so erhaltenen Ester katalytisch zu 4-(4-Isobutylphenyl)buttersäurealkylester reduziert,
c) anschließend den erhaltenen 4-(4-Isobutylphenyl)buttersäurealkylester mit einem metallorganischen Hydrid reduziert.

2) Verfahren zur Herstellung von 4-(4-Isobutylphenyl)butyraldehyd, dadurch gekennzeichnet, daß man
a) Isobutylbenzol mit Bernsteinsäuremethylesterchlorid zu 4-(4-Isobutylphenyl)-4-oxobuttersäuremethylester umsetzt,
b) den so erhaltenen Ester katalytisch zu 4-(4-Isobutylphenyl)buttersäuremethylester reduziert,
c) anschließend den erhaltenen 4-(4-Isobutylphenyl)buttersäuremethylester mit einem metallorganischen Hydrid reduziert.

3) Verfahren nach Anspruch 1 oder 2, dadurch gekennzeichnet, daß die Friedel-Crafts-Acylierung (a) in Schwefelkohlenstoff als Lösungsmittel durchgeführt wird.

4) Verfahren nach Anspruch 1 oder 2, dadurch gekennzeichnet, daß die katalytische Hydrierung der Stufe (b) in Gegenwart von Pd/Kohle erfolgt.

5) Verfahren nach Anspruch 1 oder 2, dadurch gekennzeichnet, daß als Reduktionsmittel Diisobutylaluminiumhydrid eingesetzt wird.

6) (-)-4-(2-Chlorphenyl)-2-[2-(4-isobutylphenyl)ethyl]-6,9-dimethyl-6H-thieno[3,2-f][1,2,4]-triazolo[4,3-a][1,4]-diazepin.

7) 4-(2-Chlorphenyl-2-[2-(4-isobutylphenyl)-ethyl]-9-methyl-6H-thieno[3,2-f]imidazo[1,2-a][1,4]diazepin

8) 4-(2-Chlorphenyl)-2-[2-(4-isobutylphenyl)-ethyl]-6,9-dimethyl-6H-thieno[3,2-f]imidazo[1,2-a][1,4]diazepin.

9) (-)-4-(2-Chlorphenyl)-2-[2-(4-isobutylphenyl)ethyl]-6,9-dimethyl-6H-thieno[3,2-f]imidazo[1,2-a][1, 4]-diazepin.

10) Pharmazeutische Zubereitung enthaltend (-)-4-(2-Chlorphenyl)-2-[2-(4-isobutylphenyl)ethyl]-6,9-dimethyl-6H-thieno[3,2-f][1,2,4]triazolo[4,3-a][1,4]diazepin sowie übliche Hilfs- und Trägerstoffe.